# EUROPEAN PATENT APPLICATION

(11) **EP 1 136 069 A1**
(43) Date of publication of application: **26.09.2001**
(21) Application number: 01103022.8
(22) Date of filing: 08.02.2001
(51) Int. Cl.: A61K 9/19, A61K 47/02, A61K 31/663

(54) **Pharmaceutical compositions containing clodronates for high local tolerance intramuscular administration**

(30) Priority: 21.03.2000 IT MI000584; 25.07.2000 IT MI001694
(71) Applicant: SPA SOCIETA' PRODOTTI ANTIBIOTICI S.p.A., I-20143 Milan (IT)
(72) Inventor: Mozzi, Giovanni, 20143 Milano (IT); Bonabello, Angelo, 20143 Milano (IT); Bruzzese, Tiberio, 20143 Milano (IT)
(74) Representative: Minoja, Fabrizio, Dr.

(57) **Abstract**

The preparation is described of pharmaceutical compositions containing clodronates to be administered by intramuscular route in the treatment of pathologies of the skeletal system, affording high local tolerance.

The pharmaceutical compositions described are specially characterised by suitable pH values.

## Description

This invention relates to pharmaceutical compositions containing substances belonging in the clodronate category, for the intramuscular use. The formulations of the invention, thanks to the suitable pH values, exhibit good local tolerance and are therefore easier to use in the therapeutic treatment of pathologies of the skeletal system.

Clodronates are increasingly used in numerous affections of the skeletal system, such as osteoporosis, Paget's disease (osteitis deformans), hyperparathyroidism, hypercalcemia (in particular when connected with cancer forms), ectopic ossification, treatment of bone metastases. These pathologies are becoming more frequent owing to the increasing mean age of the population and their social impact is gradually extending in view of their seriously invalidating characteristics and the potentially fatal outcome of some of them.

The pharmacological action of clodronates occurs at the skeletal system level, by inhibiting growth and dissolution of hydroxy-apatite crystals in bone and by hindering osteoclastic activity; bone absorption and consequent bone turnover are reduced.

Clodronates are usually administered by the oral, intravenous and intramuscular routes.

The oral route is the simplest and easiest from the patient's point of view. In practice, however, it is far from optimal since clodronate bioavailability is generally very low by this route, that is, 1-4% (K.Parfitt et al., Martindale - The Complete drug reference, 32^{nd} Ed., Pharmaceutical Press, London 1999, page 738). In these cases low bioavailability is offset by administration of larger doses. This means that quantities of the drug varying from 96 to 99% are lost to the pharmacological effect, while side effects, specially at gastrointestinal level (nausea and diarrhoea) intensify.

The intravenous route, presenting no problems in hospital, is scarcely applicable at home, as is the case with most osteoporotic patients.

The intramuscular route should be the route of choice as it involves no bioavailability problems and is easy to utilise even in home patients but it too presents problems as intramuscular injection of these substances is generally very painful hence scarcely accepted by the patient.

The clodronate most used in therapy is clodronate disodium, which provides water solutions with pH lower than 4.5. This is one of the reasons why the pH of clodronate parenteral solutions is always lower than 4.5. Moreover this pH minimises the risk of precipitation of alkaline-earth metal salts, present as impurities or released through hydrolysis from the glass container.

We have however found that suitable adjustment of the pH of clodronate solutions allows their administration by intramuscular injection without giving rise to the usual painful phenomena hindering at present the use of these products by injection into the muscle.

This invention relates therefore to the use of clodronates for the preparation of formulations for the intramuscular administration, which can be possible by suitable adjustment of the pH to neutral or mildly acid values, preventing the onset of severe pain usually following intramuscular injection of the clodronates. This obviates hospitalisation of the patient to undergo intravenous injection, and prevents overdosage connected to the oral administration. In addition to a reduction of the side effects of oral administration a remarkable reduction of the treatment costs is also attained.

The active substance (clodronic acid or its salts) may be used either in solutions, prevalently aqueous solutions, at concentrations varying from 5 mg/ml to 100 mg/ml (preferably 20-40 mg/ml), or in the lyophilised form, in unitary doses varying from 100 to 300 mg, for the reconstitution in a sterile solution for the parenteral use, for example water for injectable preparations.

We have surprisingly found that the lyophilised form is particularly suitable for the higher doses (f.i. > 100 mg) and for the more concentrated solutions (f.i. > 40 mg/ml), when - while working at about neutral or mildly acid pH values (pH > 4.5) - the lyophilisation is able to prevent any kind of precipitation that would occur, as shown during stability tests carried out for several months, during the shelf-life of the solution.

The extemporary dissolution of the lyophilisate gives as a rule a perfectly limpid solution.

We have also found that the same lyophilization procedure is also able to give, particularly after prolonged storage times, limpid solutions in limited volumes, such as those needed for the intramuscular administration (5 ml or lower), which could not be otherwise obtained.

The solution pH is adjusted to values of 4.5 to 8 (preferably 4.6-7.2) by adding suitable alkalinizing or buffering substances (such as sodium hydroxide, sodium carbonate, sodium bicarbonate, tris buffer, lysine). In case of lyophilised preparations, said substances will be added before carrying out the lyophilization. The formulations of the invention may also contain ordinary excipients used in pharmaceutical technique (for example, sodium chloride, p-hydroxybenzoates, benzyl alcohol). Benzyl alcohol is specially preferred.

Administration by intramuscular route can be carried out, depending of type and severity of the pathology, either daily or intermittently (that is, alternate days up to one injection every two weeks or more).

The invention is described in more detail in the following examples.

### Example 1

Formulation of an ampoule for the intramuscular administration of clodronate sodium:

| | |
|---|---|
| clodronate disodium | 100 mg |
| sodium bicarbonate q.s. to | pH 4.6 |
| water for injectable preparations q.s. to | 3.3 ml |

To prepare dissolve clodronate disodium in water; adjust the pH with a conc. solution of sodium bicarbonate; dilute to final volume with water and filter the solution through a 0.22µ membrane under sterile conditions. Distribute the resulting solution into neutral low-calcium glass ampoules under sterile conditions.

### Example 2

Formulation of an ampoule for the intramuscular administration of clodronate sodium:

| | |
|---|---|
| clodronate disodium | 100 mg |
| sodium bicarbonate q.s. to | pH 4.6 |
| water for injectable preparations q.s. to | 3.3 ml |
| benzyl alcohol | 35 mg |

For the preparation, follow the procedure described in Example 1.

### Example 3

Formulation of an ampoule for the intramuscular administration of clodronate sodium:

| | |
|---|---|
| clodronate disodium | 100 mg |
| sodium bicarbonate q.s. to | pH 5.5 |
| water for injectable preparations q.s. to | 3.3 ml |

For the preparation, follow the procedure described in Example 1.

### Example 4

Formulation of an ampoule for the intramuscular administration of clodronate sodium:

| | |
|---|---|
| clodronate disodium | 100 mg |
| tris buffer q.s. to | pH 7 |
| water for injectable preparations q.s. to | 5 ml |

For the preparation, follow the procedure described in Example 1 and distribute the resulting solution into neutral glass ampoules under sterile conditions.

### Example 5

Formulation of an ampoule for the intramuscular administration of clodronic acid:

| | |
|---|---|
| clodronic acid | 150 mg |
| sodium hydroxide q.s. to | pH 8 |
| water for injectable preparations q.s. to | 5 ml |

For the preparation, follow the procedure described in Example 1 and distribute the resulting solution into neutral glass ampoules under sterile conditions.

### Example 6

Formulation of an ampoule for the intramuscular administration of clodronate sodium:

| | | |
|---|---|---|
| Lyophilizate | clodronate disodium | 200 mg |
| | sodium bicarbonate q.s. to | pH 5.5 |
| | water for injectable preparations q.s. to | 3 ml |
| Solvent vial | water for injectable preparations q.s. to | 3 ml |

To prepare dissolve clodronate disodium in water; adjust pH with a sodium bicarbonate concentrated solution; dilute to final volume with water; filter the solution through a 0.22 µ membrane under sterile conditions; distribute the resulting solution into neutral glass ampoules and lyophilise under sterile conditions. The lyophilised preparation will be reconstituted before use with the solvent vial.

### Example 7

Formulation of an ampoule for the intramuscular administration of clodronate sodium:

| | | |
|---|---|---|
| Lyophilizate | clodronate disodium | 200 mg |
| | sodium bicarbonate q.s. to | pH 5.5 |
| | water for injectable preparations q.s. to | 5 ml |
| Solvent vial | water for injectable preparations q.s. to | 5 ml |

For the preparation, follow the procedure described in Example 6.

### Example 8

Formulation of an ampoule for the intramuscular administration of clodronate sodium:

| | | |
|---|---|---|
| Lyophilizate | clodronate disodium | 200 mg |
| | sodium bicarbonate q.s. to | pH 7 |
| | water for injectable preparations q.s. to | 5 ml |
| Solvent vial | water for injectable preparations q.s. to | 5 ml |

For the preparation, follow the procedure described in Example 6.

### Example 9

Formulation of an ampoule for the intramuscular administration of clodronate sodium:

| | | |
|---|---|---|
| Lyophilizate | clodronate disodium | 200 mg |
| | sodium bicarbonate q.s. to | pH 5.5 |
| | water for injectable preparations q.s. to | 5 ml |
| Solvent vial | benzyl alcohol | 50 mg |
| | water for injectable preparations q.s. to | 5 ml |

For the preparation, follow the procedure described in Example 6.

### Example 10

Formulation of an ampoule for the intramuscular administration of clodronate sodium:

| | | |
|---|---|---|
| Lyophilizate | clodronate disodium | 200 mg |
| | Polivinylpirrolidone | 200 mg |
| | Sodium hydroxide q.s. to | pH 5.5 |
| | water for injectable preparations q.s. to | 5 ml |
| Solvent vial | benzyl alcohol | 120 mg |
| | water for injectable preparations q.s. to | 4 ml |

For the preparation, follow the procedure described in Example 6.

### Example 11

Formulation of an ampoule for the intramuscular administration of clodronate sodium:

| | | |
|---|---|---|
| Lyophilizate | clodronate disodium | 100 mg |
| | sodium bicarbonate q.s. to | pH 5.5 |
| | water for injectable preparations q.s. to | 2 ml |
| Solvent vial | water for injectable preparations q.s. to | 3 ml |

For the preparation, follow the procedure described in Example 6.

### Example 12

Formulation of an ampoule for the intramuscular administration of clodronate sodium:

| | | |
|---|---|---|
| Lyophilizate | clodronate disodium | 300 mg |
| | sodium bicarbonate q.s. to | pH 5.5 |
| | water for injectable preparations q.s. to | 5 ml |
| Solvent vial | benzyl alcohol | 50 mg |
| | water for injectable preparations q.s. to | 5 ml |

For the preparation, follow the procedure described in Example 6.

### Example 13

The pain effect was tested by the licking test in rabbits, of sodium clodronate preparations having different pH.

The test was performed in 8 New Zealand male albino rabbits weighing 3.2 to 3.7 kg. Before treatment their dorsal area was shaven.

Treatment consisted of intradermal injection of 200 µl of the test solution into the animal dorsal region. Each animal used in the test was treated with preparations at several pH values, injected into different dorsal areas at 48-hour intervals.

Tolerance to the pain caused by the test solutions was inversely proportional to the number of responses of the animal, reacting to discomfort or pain by licking the portion of skin injected.

The animals were kept under observation for 30 minutes to determine the responses to pain; the injection spot was controlled after 24 and 48 hours.

The following preparations were tested:

| | Preparation A | Preparation B | Preparation C |
|---|---|---|---|
| Clodronate disodium | 3 g | 3 g | 3 g |
| 1N sodium hydroxide q.s. to | pH 4.2 | pH 5.5 | pH 7.0 |
| Water q.s. to | 100 ml | 100 ml | 100 ml |

The following table reports the results obtained, statistically elaborated through the Student's paired t test:

| Response to pain stimulation tested by the licking test in rabbits | | | |
|---|---|---|---|
| No. of responses to stimulation by various preparations | | | |
| Animal | Preparation A | Preparation B | Preparation C |
| 1 | 3 | 1 | 0 |
| 2 | 9 | 5 | 4 |
| 3 | 12 | 4 | 3 |
| 4 | 2 | 2 | 1 |
| 5 | 5 | 3 | 1 |
| 6 | 10 | 8 | 8 |
| 7 | 7 | 4 | 2 |
| 8 | 2 | 2 | 2 |
| Mean values (no. of resp./animal) | 6.3 | 3.6 | 2.6 |
| P (against A) | - | <0.05 | <0.01 |

It is evident that tolerance to preparations B and C (pH 5.5 and 7) is definitely and significantly better than tolerance to solution A (pH 4.2). Worth mentioning is the fact that, 24 hours after each treatment, at the site of intradermal injection with preparation A (pH 4.2), mild oedema and reddening were still present, which decreased in the next 24 hours. Administration of preparations B and C (pH 5.5 and 7) caused no change at the injection site controlled 24 hours after treatment.

### Example 14

The pain effect of clodronic acid preparations having different pH was tested by the licking test in rabbits.

The test was performed in 4 New Zealand male albino rabbits weighing 2.5 to 2.7 kg of body weight. Before treatment their dorsal area was shaven.

Treatment consisted of intradermal injection of 200 µl of the test solution into the animal dorsal region. Each animal used in the test was treated with preparations at several pH values, injected into different dorsal areas at 48-hour intervals.

Tolerance to the pain caused by the test solutions was inversely proportional to the number of responses of the animal, reacting to discomfort or pain by licking the portion of skin injected.

The following preparations were tested:

| | Preparation A | Preparation B | Preparation C |
|---|---|---|---|
| Clodronate disodium | 3 g | 3 g | 3 g |
| Tris buffer q.s. to | pH 4.2 | pH 7 | pH 9 |
| Water q.s. to | 100 ml | 100 ml | 100 ml |

The following table reports the results obtained, statistically elaborated through the Student's paired t test:

| Response to pain stimulation tested by the licking test in rabbits | | | |
|---|---|---|---|
| No. of responses to stimulation by various preparations | | | |
| Animal | Preparation A | Preparation B | Preparation C |
| 1 | 8 | 0 | 5 |
| 2 | 0 | 0 | 0 |
| 3 | 8 | 1 | 5 |
| 4 | 6 | 2 | 6 |
| Mean values (no. of resp./animal) | 5.5 | 0.8 | 4.0 |
| P (against A) | - | <0.1 | n.s. |
| P (against C) | n.s. | <0.1 | - |

It is evident that tolerance to preparation B (pH 7.0) is definitely and significantly better than tolerance to solutions A and C (pH 4.2 and 9), not differing significantly from each other.

## Claims

1. Pharmaceutical compositions for intramuscular injection, containing substances of the clodronate group, **characterised by** about neutral or mildly acid pH.

2. Pharmaceutical compositions according to claim 1 wherein said substances in question are clodronic acid or a pharmaceutically acceptable salt thereof.

3. Pharmaceutical compositions according to claim 1 wherein the pharmaceutical substance is clodronate disodium.

4. Pharmaceutical compositions according to any of claims 1 to 3 having pH from 4.5 to 8.

5. Pharmaceutical compositions according to claim 4 having pH from 4.6 to 7.2.

6. Pharmaceutical compositions according to claims 1 to 5 in form of solution ready to use.

7. Pharmaceutical compositions according to claims 1 - 5 wherein the concentration of the clodronates in solution varies from 5 mg/ml to 100 mg/ml.

8. Pharmaceutical compositions according to claim 7 wherein the clodronate concentration varies from 20 mg/ml to 40 mg/ml.

9. Pharmaceutical compositions according to any one of claims 1 to 5, wherein the active substance is in lyophilised form for the reconstitution in a sterile solution.

10. Pharmaceutical compositions according to claim 9, comprising 100 to 300 mg of active substance.

11. Pharmaceutical compositions according to claims 1 to 10, containing excipients useful to the pharmaceutical formulation.

12. Pharmaceutical compositions according to claim 11 wherein one of the excipients is benzyl alcohol.

13. Pharmaceutical compositions according to any one of claims 11 and 12 containing buffering or alkalinizing substances.
